# EUROPEAN PATENT APPLICATION

(11) **EP 3 950 925 A1**
(43) Date of publication of application: **09.02.2022**
(21) Application number: 20785419.1
(22) Date of filing: 27.03.2020
(51) Int. Cl.: C12M 3/00, C12N 5/07, C12N 5/0735, C12N 5/074, C12N 1/00

(54) **CELL CULTURE SCAFFOLD MATERIAL, CELL CULTURE VESSEL, CELL CULTURE CARRIER, CELL CULTURE FIBER AND CELL CULTURE METHOD**

(30) Priority: 29.03.2019 JP 2019068407
(71) Applicant: SEKISUI CHEMICAL CO., LTD., Osaka-shi Osaka 530-8565 (JP)
(72) Inventor: HANEDA, Satoshi, Mishima-gun, Osaka 618-0021 (JP); MANABE, Yuriko, Mishima-gun, Osaka 618-0021 (JP); ISHII, Ryoma, Mishima-gun, Osaka 618-0021 (JP); IGUCHI, Hiroki, Mishima-gun, Osaka 618-0021 (JP); YAMAUCHI, Hiroshi, Mishima-gun, Osaka 618-0021 (JP); OOMURA, Takahiro, Hasuda-shi, Saitama 349-0198 (JP)
(74) Representative: Ter Meer Steinmeister & Partner
(86) International application number: PCT/JP2020/014014
(87) International publication number: WO 2020/203770

(57) **Abstract**

Provided is a scaffolding material for cell culture having suitable hydrophilicity and strength, high fixation of cells after seeding, and highly efficient cell proliferation. A scaffolding material for cell culture according to the present invention is one containing a synthetic resin, and having a nitrogen content of the synthetic resin of 0.1% by mass or more and 10% by mass or less.

## Description

### TECHNICAL FIELD

The present invention relates to a scaffolding material for cell culture, used for culturing a cell. The present invention also relates to a container for cell culture, a carrier for cell culture, a fiber for cell culture and a cell culture method obtained by or conducted by using the scaffolding material for cell culture.

### BACKGROUND ART

Animal cells such as human, mouse, rat, pig, cattle and monkey cells are used in research and development in academic fields, drug development fields, regenerative medicine fields and the like. As scaffold materials used for culturing an animal cell, adhesive proteins such as laminin and vitronectin, and natural polymer materials such as Matrigel derived from mouse sarcoma are used.

Furthermore, as shown in Patent Documents 1 to 3 below, scaffold materials obtained by using a synthetic resin are also known.

Patent Document 1 below discloses a carrier for cell culture composed of a molded product made of a polyvinyl acetal compound or a molded product made of the polyvinyl acetal compound and a water-soluble polysaccharide, having a degree of acetalization of the polyvinyl acetal compound of 20 to 60 mol%.

Patent Document 2 below discloses a scaffold material made of hydrogel, for culturing an undifferentiated cell in an undifferentiated state.

Furthermore, Patent Document 3 below discloses a cell culture method by which pluripotent stem cells are maintained in an undifferentiated state, including a step of culturing the pluripotent stem cells on an incubator having a surface coated with a polyrotaxane block copolymer.

### Related Art Documents

### Patent Documents

Patent Document 1: JP 2006-314285 A
Patent Document 2: JP 2010-158180 A
Patent Document 3: JP 2017-23008 A

### SUMMARY OF THE INVENTION

### PROBLEMS TO BE SOLVED BY THE INVENTION

By using natural polymer materials as scaffold materials, the fixation of cells after seeding can be enhanced. However, natural polymer materials are expensive, have large variations between lots because they are naturally occurring substances, and have safety concerns due to animal-derived components.

On the other hand, scaffold materials obtained by using a synthetic resin as described in Patent Documents 1 to 3 are cheaper, have less variation between lots, and are excellent in safety as compared with scaffold materials obtained by using a natural polymer material. However, the scaffold materials obtained by using a synthetic resin as described in Patent Documents 1 to 3 are extremely hydrophilic. Therefore, the scaffold materials are swelled by a liquid medium so that the scaffold materials are peeled off etc. In this way, the strength may be inferior. In addition, in the scaffold materials obtained by using a synthetic resin as described in Patent Documents 1 to 3, the fixation of cells after seeding is so low that the cells do not proliferate sufficiently.

Conventionally, it has been difficult to prepare a scaffolding material for cell culture which can have suitable hydrophilicity and strength, high fixation rate of cells after seeding, and highly efficient cell proliferation.

An object of the present invention is to provide a scaffolding material for cell culture having suitable hydrophilicity and strength, high fixation of cells after seeding, and highly efficient cell proliferation. Another object of the present invention is to provide a container for cell culture, a carrier for cell culture, a fiber for cell culture and a cell culture method obtained by or conducted by using the scaffolding material for cell culture.

### MEANS FOR SOLVING THE PROBLEMS

According to a broad aspect of the present invention, a scaffolding material for cell culture containing a synthetic resin is provided, having a nitrogen content of the synthetic resin of 0.1% by mass or more and 10% by mass or less.

In a certain aspect of the scaffolding material for cell culture according to the present invention, the synthetic resin includes a Bronsted basic group in an amount of 1 mol% or more and 50 mol% or less.

In another certain aspect of the scaffolding material for cell culture according to the present invention, the Bronsted basic group is an amine-based basic group.

In yet another certain aspect of the scaffolding material for cell culture according to the present invention, the synthetic resin includes at least one structural unit selected from the group consisting of a structural unit having an amine structure, a structural unit having an imine structure and a structural unit having an amide structure.

In yet another certain aspect of the scaffolding material for cell culture according to the present invention, the synthetic resin includes a structural unit having an amide structure.

In yet another certain aspect of the scaffolding material for cell culture according to the present invention, the synthetic resin includes both a structural unit having an imine structure and a structural unit having an amine structure.

In yet another certain aspect of the scaffolding material for cell culture according to the present invention, the cell is a somatic cell, a stem cell, a progenitor cell or a differentiated cell.

According to a broad aspect of the present invention, a container for cell culture is provided, including the scaffolding material for cell culture.

According to a broad aspect of the present invention, a carrier for cell culture is provided, containing the scaffolding material for cell culture and a polysaccharide.

According to a broad aspect of the present invention, a fiber for cell culture is provided, including the scaffolding material for cell culture.

According to a broad aspect of the present invention, a cell culture method is provided, including using the scaffolding material for cell culture.

### EFFECT OF THE INVENTION

According to the present invention, there are provided a scaffolding material for cell culture having suitable hydrophilicity and strength, high fixation of cells after seeding, and highly efficient cell proliferation, and a container for cell culture, a carrier for cell culture, a fiber for cell culture and a cell culture method obtained by or conducted by using the scaffolding material for cell culture.

### BRIEF DESCRIPTION OF DRAWINGS

[Fig. 1] Fig. 1A is a schematic perspective view of the container for cell culture according to an embodiment, and Fig. 1B is a schematic sectional side view thereof.
[Fig. 2] Fig. 2 is a schematic perspective view of the 6-well type container for cell culture according to an embodiment.

### MODES FOR CARRYING OUT THE INVENTION

Hereinafter, a description is made of the present invention with reference to embodiments, but the present invention is not limited to the following embodiments.

### [Scaffolding material for cell culture]

A scaffolding material for cell culture according to the present invention is one containing a synthetic resin, and having a nitrogen content of the synthetic resin of 0.1% by mass or more and 10% by mass or less.

As a result of intensive studies, the present inventors have found that the above problems can be solved by using a synthetic resin having a nitrogen content of 0.1% by mass or more and 10% by mass or less, and thus have completed the present invention.

From the viewpoint of more effectively exerting the effect of the present invention, the nitrogen content of the synthetic resin is preferably 0.3% by mass or more, more preferably 0.5% by mass or more, and preferably 8% by mass or less, more preferably 5% by mass or less.

The elemental composition of the synthetic resin can be analyzed by X-ray photoelectron spectroscopy. Examples of the device used in the X-ray photoelectron spectroscopy include a "multifunctional scanning X-ray photoelectron spectrometer (XPS)" manufactured by ULVAC-PHI, Inc. The total amount of all the detected elements is defined as 100%, and the occupation rate (%) of nitrogen in all the elements is defined as the nitrogen content.

The scaffolding material for cell culture according to the present invention includes an aspect in which the material is composed of only a synthetic resin having a nitrogen content of 0.1% by mass or more and 10% by mass or less.

The scaffolding material for cell culture according to the present invention has so suitable hydrophilicity and strength that the fixation of cells after seeding is improved. In particular, in a serum-free medium culture containing no feeder cell or adhesive protein, the initial fixation rate of cells after seeding is improved. The scaffolding material for cell culture is not particularly limited for component as long as the above-mentioned requirements are satisfied.

The synthetic resin refers to a resin mainly composed of a polymer (hereinafter, also simply referred to as "polymer") obtained by polymerizing (including polycondensing) a polymerizable monomer (hereinafter, also simply referred to as "monomer"). The polymer may be a homopolymer obtained by polymerizing only one type of monomer, or a copolymer obtained by polymerizing two or more types of monomers.

Examples of the polymer include a polymer composed of one or more polymerizable monomers of saturated or unsaturated hydrocarbons, aromatic hydrocarbons, saturated or unsaturated fatty acids, aromatic carboxylic acids, saturated or unsaturated ketones, aromatic ketones, saturated or unsaturated alcohols, aromatic alcohols, saturated or unsaturated amines, aromatic amines, saturated or unsaturated thiols, aromatic thiols and organosilicon compounds.

Specific examples of the polymer include polyolefin, polyether, polyvinyl alcohol, a polyvinyl acetal resin, polyester, poly(meth)acrylic ester, an epoxy resin, polyamide, polyimide, polyurethane, polycarbonate, cellulose and polypeptide.

From the viewpoint of further enhancing the fixation of cells, the synthetic polymer (polymer) is preferably a poly(meth)acrylic ester or a polyvinyl acetal resin, and a polyvinyl acetal resin is more preferable.

The polymers may be used alone or in combination of two or more. When two or more polymers are combined, they may be used as a mixture, or may be used as a polymer in which the skeletons of the two or more polymers are chemically bonded. Accordingly, when a plurality of them are combined as a synthetic resin, it is preferable to combine poly(meth)acrylic ester and polyvinyl acetal.

In the present specification, "(meth)acrylate" refers to at least one selected from the group consisting of (meth)acrylic ester and (meth)acrylic acid. In addition, poly(meth)acrylate is not only polymers obtained by polymerizing a monomer, (meth)acrylic ester or (meth)acrylic acid, but also includes those obtained by copolymerizing a monomer in addition to (meth)acrylic ester or (meth)acrylic acid.

The (meth)acrylic ester is not particularly limited, but preferably contains at least one selected from the group consisting of alkyl (meth)acrylic esters, cyclic alkyl (meth)acrylic esters, aryl (meth)acrylic esters, (meth)acrylamides, polyethylene glycol (meth)acrylates and phosphorylcholine (meth)acrylates.

Examples of the alkyl (meth)acrylic ester include methyl (meth)acrylate, ethyl (meth)acrylate, n-propyl (meth)acrylate, isopropyl (meth)acrylate, n-butyl (meth)acrylate, isobutyl (meth)acrylate, t-butyl (meth)acrylate, n-octyl (meth)acrylate, isooctyl (meth)acrylate, 2-ethylhexyl (meth)acrylate, nonyl (meth)acrylate, isononyl (meth)acrylate, decyl (meth)acrylate, isodecyl (meth)acrylate, lauryl (meth)acrylate, stearyl (meth)acrylate and isotetradecyl (meth)acrylate.

These alkyl (meth)acrylic esters are not particularly limited, but may be substituted with various substituents including an alkoxy group having 1 to 3 carbon atoms and a tetrahydrofurfuryl group. Examples of such alkyl (meth)acrylic esters include methoxyethyl acrylate and tetrahydrofurfuryl acrylate.

Examples of the cyclic alkyl (meth)acrylic ester include cyclohexyl (meth)acrylate and isobornyl (meth)acrylate.

Examples of the aryl (meth)acrylic ester include phenyl (meth)acrylate and benzyl (meth)acrylate.

Examples of the acrylamide include (meth)acrylamide, N-isopropyl (meth)acrylamide, N-tert-butyl (meth)acrylamide, N,N'-dimethyl (meth)acrylamide, (3-(meth)acrylamidopropyl) trimethylammonium chloride, 4-(meth)acryloylmorpholine, 3-(meth)acryloyl-2-oxazolidinone, N-[3-(dimethylamino) propyl] (meth)acrylamide, N-(2-hydroxyethyl) (meth)acrylamide, N-methylol (meth)acrylamide and 6-(meth)acrylamidohexanoic acid.

Examples of the polyethylene glycol (meth)acrylate include methoxy-polyethylene glycol (meth)acrylate, ethoxy-polyethylene glycol (meth)acrylate, hydroxy-polyethylene glycol (meth)acrylate, methoxy-diethylene glycol (meth)acrylate, ethoxy-diethylene glycol (meth)acrylate, hydroxy-diethylene glycol (meth)acrylate, methoxytriethylene glycol (meth)acrylate, ethoxy-triethylene glycol (meth)acrylate and hydroxy-triethylene glycol (meth)acrylate.

Examples of the phosphorylcholine (meth)acrylate include 2-(meth)acryloyloxyethyl phosphorylcholine.

Monomers other than the (meth)acrylic esters are not particularly limited, but include (meth)acrylic acids, ethylene and vinyl esters.

The (meth) acrylic esters may be used alone or in combination of two or more.

Note that, in this specification, "(meth)acrylic" means "acrylic" or "methacrylic", and "(meth)acrylate" means "acrylate" or "methacrylate".

Among the synthetic resins, it is preferable to use a polyvinyl acetal resin. Hereinafter, a description is made of the polyvinyl acetal resin.

### (Polyvinyl acetal resin)

The polyvinyl acetal resin can be synthesized by acetalizing polyvinyl alcohol with an aldehyde. The polyvinyl acetal resin has an acetyl group, a hydroxyl group and an acetal group on the side chain.

The polyvinyl acetal resin may be a polyvinyl acetal resin (hereinafter, also referred to as a modified polyvinyl acetal resin) having another substituent (a substituent other than an acetyl group, a hydroxyl group and an acetal group) on the side chain. As the other substituent, a substituent having a Bronsted basic group described below is preferable.

The aldehyde used for acetalizing polyvinyl alcohol is not particularly limited. Examples of the aldehyde include aldehydes having 1 to 10 carbon atoms. The aldehyde may have a chain aliphatic group, a cyclic aliphatic group or an aromatic group. The aldehyde may be a chain aldehyde or a cyclic aldehyde.

The type of the aldehyde includes formaldehyde, acetaldehyde, propionaldehyde, butyraldehyde, pentanal, hexanal, heptanal, octanal, nonanal, decanal, acrolein, benzaldehyde, cinnamaldehyde, perylaldehyde, formylpyridine, formylimidazole, formylpyrrole, formylpiperidine, formyltriazole, formyltetrazole, formylindole, formylisoindole, formylpurine, formylbenzimidazole, formylbenzotriazole, formylquinoline, formylisoquinoline, formylquinoxaline, formylcinnoline, formylpteridine, formylfuran, formyloxolane, formyloxane, formylthiophene, formylthiolane, formylthiane, formyladenine, formylguanine, formylcytosine, formylthymine and formyluracil. The aldehyde may be used alone or in combination of two or more.

The aldehyde is preferably formaldehyde, acetaldehyde, propionaldehyde, butyraldehyde or pentanal, more preferably butyraldehyde. Accordingly, the polyvinyl acetal resin is more preferably a polyvinyl butyral resin.

The addition amount of the aldehyde can be appropriately set according to the desired amount of the acetal group. In particular, it is preferable that the addition amount of the aldehyde is 60 to 95 mol%, preferably 65 to 90 mol% with respect to 100 mol% polyvinyl alcohol, because the acetalization reaction is efficiently performed and unreacted aldehydes are easily removed.

The polyvinyl acetal resin may be a resin in which a vinyl compound is copolymerized. The polyvinyl acetal resin may be a copolymer with a vinyl compound. In the present invention, as the polyvinyl acetal resin, a polyvinyl acetal resin obtained by copolymerization with a vinyl group is also considered. The vinyl compound is a compound having a vinyl group (H₂C=CH-). The vinyl compound may be a polymer having a constitutional unit having a vinyl group.

The copolymer may be a block copolymer of a polyvinyl acetal resin and a vinyl compound, or a graft copolymer in which a vinyl compound is grafted to a polyvinyl acetal resin. The copolymer is preferably a graft copolymer.

The copolymer can be synthesized, for example, by any of the following methods (1) to (3) . (1) A method for synthesizing a polyvinyl acetal resin including using polyvinyl alcohol in which a vinyl compound is copolymerized. (2) A method for synthesizing a polyvinyl acetal resin including using polyvinyl alcohol and polyvinyl alcohol in which a vinyl compound is copolymerized. (3) A method including graft-copolymerizing a vinyl compound to a pre-graft-copolymerized polyvinyl acetal resin.

The vinyl compound includes ethylene, vinylamine, allylamine, vinylpyrrolidone, maleic anhydride, maleimide, itaconic acid and (meth)acrylic acids. The vinyl compound may be used alone or in combination of two or more.

For the (meth)acrylic acids, the (meth)acrylic ester is not particularly limited, but preferably contains at least one selected from alkyl (meth)acrylic esters, cyclic alkyl (meth)acrylic esters, aryl (meth)acrylic esters, (meth)acrylamides, polyethylene glycol (meth)acrylates and phosphorylcholine (meth)acrylates.

Examples of the alkyl (meth)acrylic ester include methyl (meth)acrylate, ethyl (meth)acrylate, n-propyl (meth)acrylate, isopropyl (meth)acrylate, n-butyl (meth)acrylate, isobutyl (meth)acrylate, t-butyl (meth)acrylate, n-octyl (meth)acrylate, isooctyl (meth)acrylate, 2-ethylhexyl (meth)acrylate, nonyl (meth)acrylate, isononyl (meth)acrylate, decyl (meth)acrylate, isodecyl (meth)acrylate, lauryl (meth)acrylate, stearyl (meth)acrylate and isotetradecyl (meth)acrylate.

Examples of the cyclic alkyl (meth)acrylic ester include cyclohexyl (meth)acrylate and isobornyl (meth)acrylate.

Examples of the aryl (meth)acrylic ester include phenyl (meth)acrylate and benzyl (meth)acrylate.

Examples of the acrylamide include (meth)acrylamide, N-isopropyl (meth)acrylamide, N-tert-butyl (meth)acrylamide, N,N'-dimethyl (meth)acrylamide, (3-(meth)acrylamidopropyl) trimethylammonium chloride, 4-(meth)acryloylmorpholine, 3-(meth)acryloyl-2-oxazolidinone, N-[3-(dimethylamino) propyl] (meth)acrylamide, N-(2-hydroxyethyl) (meth)acrylamide, N-methylol (meth)acrylamide and 6-(meth)acrylamidohexanoic acid.

Examples of the polyethylene glycol (meth)acrylate include methoxy-polyethylene glycol (meth)acrylate, ethoxy-polyethylene glycol (meth)acrylate, hydroxy-polyethylene glycol (meth)acrylate, methoxy-diethylene glycol (meth)acrylate, ethoxy-diethylene glycol (meth)acrylate, hydroxy-diethylene glycol (meth)acrylate, methoxytriethylene glycol (meth)acrylate, ethoxy-triethylene glycol (meth)acrylate and hydroxy-triethylene glycol (meth)acrylate.

Examples of the phosphorylcholine (meth)acrylate include 2-(meth)acryloyloxyethyl phosphorylcholine.

The (meth) acrylic acids may be used alone or in combination of two or more.

The synthetic resin preferably has in a part thereof a Bronsted basic group. In this case, in a serum-free medium culture containing no feeder cell or adhesive protein, the initial fixation rate of cells after seeding is improved, and cell culture is easily performed.

From the viewpoint of more effectively exerting the effect of the present invention, the synthetic resin preferably contains a Bronsted basic group in an amount of 1 mol% or more and 50 mol% or less, more preferably 1 mol% or more and 30 mol% or less.

The Bronsted basic group is a generic term for a functional group that can receive a hydrogen ion H⁺ from another substance. Examples of the Bronsted basic group include amine-based basic groups such as a substituent having an amine structure, a substituent having an imine structure, a substituent having an amide structure and a substituent having an imide structure.

Accordingly, the synthetic resin preferably contains at least one structural unit selected from the group consisting of a structural unit having an imine structure, a structural unit having an amine structure, a structural unit having an amide structure and a structural unit having an imide structure. The synthetic resin preferably contains at least one structural unit selected from the group consisting of a structural unit having an imine structure, a structural unit having an amine structure and a structural unit having an amide structure.

The synthetic resin preferably includes both of a structural unit having an imine structure and a structural unit having an amine structure.

In the present invention, the imine structure refers to a structure having a C=N bond. The synthetic resin preferably has an imine structure on the side chain. In addition, the imine structure may be directly bonded to a carbon atom constituting the main chain of the synthetic resin, or may be bonded to the main chain via a linking group such as an alkylene group. Note that having the imine structure on the side chain includes having the imine structure on the graft chain of the synthetic resin. Examples of the structural unit having an imine structure include a structural unit represented by the following formula (11) or (12).

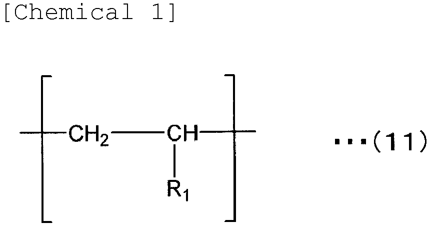

In the formula (11), R₁ represents a group having an imine structure.

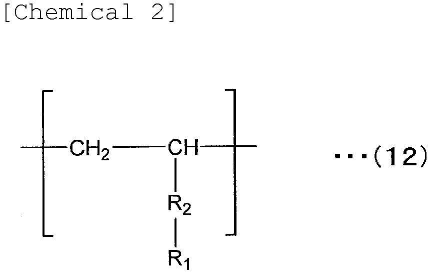

In the formula (12), R₁ represents a group having an imine structure, and R₂ represents an alkylene group. The alkylene group has preferably 1 or more, and preferably 12 or less, more preferably 5 or less carbon atoms. When the carbon number of the alkylene group is equal to or more than the above-mentioned lower limit and equal to or less than the above-mentioned upper limit, the strength of the scaffolding material for cell culture can be enhanced.

The alkylene group includes linear alkylene groups such as a methylene group, ethylene group, trimethylene group, tetramethylene group, pentamethylene group, hexamethylene group, octamethylene group and decamethylene group, branched alkylene groups such as a methyl methylene group, methylethylene group, 1-methylpentylene group and 1,4-dimethylbutylene group, and cyclic alkylene groups such as a cyclopropylene group, cyclobutylene group and cyclohexylene group. The alkylene group is preferably a linear alkyl group such as a methylene group, ethylene group, trimethylene group or tetramethylene group, more preferably a methylene group or ethylene group.

R₁ in the formula (11) and R₁ in the formula (12) include groups represented by the following formula (13).

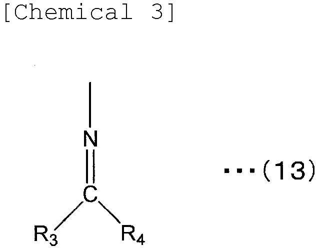

In the formula (13), R₃ represents a hydrogen atom or a hydrocarbon group having 1 to 18 carbon atoms, and R₄ represents a hydrocarbon group having 1 to 18 carbon atoms.

The hydrocarbon group includes a saturated hydrocarbon group, an unsaturated hydrocarbon group and an aromatic hydrocarbon group. The hydrocarbon group may be one composed of only any one of a saturated hydrocarbon group, an unsaturated hydrocarbon group and an aromatic hydrocarbon group, or one in which two or more of them are used.

The saturated hydrocarbon group includes methyl, ethyl, n-propyl, isopropyl, n-butyl, iso-butyl, sec-butyl, tert-butyl, pentyl, hexyl, heptyl, 2-ethylhexyl, octyl, nonyl, decyl, undecyl, dodecyl, tridecyl, tetradecyl, pentadecyl and octadecyl groups. The saturated hydrocarbon group is preferably a methyl group, an ethyl group, an n-propyl group or an n-butyl group.

The aromatic hydrocarbon group includes a phenyl group, toluyl group, xylyl group, t-butylphenyl group and benzyl group.

It is preferable that the structural unit having an imine structure be a structural unit having the structure represented by the formula (11), and in the formula (13), R₃ be a hydrogen atom, a methyl group or an ethyl group, and R₄ be a methyl group, an ethyl group or a propyl group.

In the synthetic resin, the content of the structural unit having an imine structure is preferably 0.1 mol% or more, more preferably 1.0 mol% or more, and preferably 20.0 mol% or less, more preferably 15.0 mol% or less. When the content is equal to or more than the above-mentioned lower limit, the viscosity stability over time can be better. When the content is equal to or less than the above-mentioned upper limit, acetalization can be sufficiently advanced.

When a polyvinyl acetal resin (modified polyvinyl acetal resin) is used as the synthetic resin, the ratio of the content of the structural unit having an imine structure to the degree of acetalization mentioned below (the content of the structural unit having an imine structure/degree of acetalization) is preferably 0.001 or more, preferably 0.5 or less. When the ratio (the content of the structural unit having an imine structure/degree of acetalization) is equal to or more than the above-mentioned lower limit and equal to or less than the above-mentioned upper limit, high strength and excellent adhesiveness can be achieved at the same time, and the durability after adhesion can be improved.

The synthetic resin preferably has a structural unit having an amine structure or a structural unit having an amide structure.

The synthetic resin preferably has the amine structure or the amide structure on the side chain. In addition, the amine structure or the amide structure may be directly bonded to a carbon atom constituting the main chain of the synthetic resin, or may be bonded to the main chain via a linking group such as an alkylene group. Furthermore, the amine group in the amine structure may be a primary amine group, a secondary amine group, a tertiary amine group or a quaternary amine group. Among them, a primary amine group is preferable from the viewpoint of enhancing the fixation of cells.

Note that having the amine structure or amide structure on the side chain means having the amine structure or the amide structure on the graft chain of the synthetic resin.

In particular, the amine structure is preferably -NH₂. In the present invention, the amide structure refers to a structure having -C(=O)-NH-. In particular, the structural unit having the amine structure preferably is a structure represented by the following formula (21). In addition, the structural unit having the amide structure preferably has a structure represented by the following formula (31).

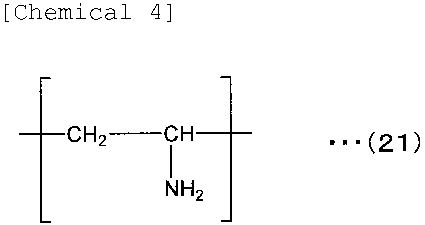

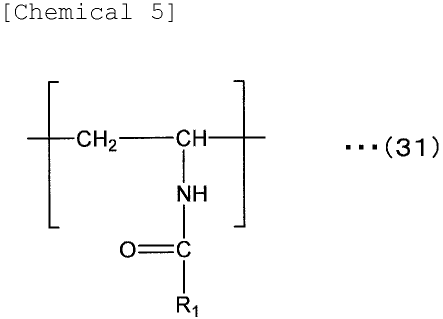

In the formula (31), R₁ represents a hydrogen atom or a hydrocarbon group having 1 to 10 carbon atoms. The hydrocarbon group includes an alkyl group, an alkenyl group, a cycloalkyl group and a cycloalkenyl group.

In the synthetic resin, the content of the structural unit having the amine structure or the amide structure is preferably 0.1 mol% or more, more preferably 0.5 mol% or more, and preferably 20 mol% or less, more preferably 10 mol% or less. When the content is equal to or more than the above-mentioned lower limit, additional properties can be improved. When the content is equal to or less than the above-mentioned upper limit, the solubility is not so excessively increased that the synthetic resin powder (for example, modified polyvinyl acetal resin powder) can be easily taken out by precipitation method.

In the synthetic resin, the total content of the structural unit having the amine structure or the amide structure, and the structural unit having an imine structure is preferably 0.1 mol% or more, more preferably 0.5 mol% or more, and preferably 20 mol% or less, more preferably 10 mol% or less.

In the synthetic resin, when both of the imine structure and the amine structure or the amide structure are contained, the ratio of the content of the structural unit having an imine structure to the content of the structural unit having an amine structure or an amide structure (the structural unit having an imine structure/the structural unit having an amine structure or an amide structure) is preferably 0.5/99.5 to 99.5/0.5. When the ratio is 0.5/99.5 or more, the viscosity stability over time can be sufficient, whereas when the above ratio is 99.5/0.5 or less, the crosslinking performance can be sufficiently exhibited from the viewpoint of the fixation of cells. The ratio (the structural unit having an imine structure/the structural unit having an amine structure or an amide structure) is preferably 5/95 or more and preferably 75/25 or less.

The synthetic resin preferably contains at least one structural unit selected from the group consisting of a structural unit having an amine structure, a structural unit having an imine structure and a structural unit having an amide structure.

The synthetic resin preferably includes both of a structural unit having an imine structure and a structural unit having an amine structure.

The content of the structural unit having an imine structure, the content of the structural unit having an imide structure, the content of the structural unit having an amine structure and the content of the structural unit having an amide structure can be measured by ¹H-NMR (nuclear magnetic resonance spectrum).

When a polyvinyl acetal resin is used as the synthetic resin, the degree of acetalization of the polyvinyl acetal resin (degree of butyralization in the case of the polyvinyl butyral resin) is not particularly limited, but is preferably 54 mol% or more, more preferably 60 mol% or more, still more preferably 65 mol% or more, and preferably 90 mol% or less, more preferably 85 mol% or less. When the degree of acetalization is equal to or higher than the above-mentioned lower limit, the fixation of cells is excellent, and thus cell proliferation can be performed with high efficiency. When the degree of acetalization is equal to or lower than the above-mentioned upper limit, the solubility in solvent can be better.

The method for adjusting the degree of acetalization to exceed the above-mentioned lower limit value is not particularly limited, but adding an excess amount of the aldehyde in the acetalization reaction allows the acetalization reaction to proceed sufficiently.

When a polyvinyl acetal resin is used as the synthetic resin, the degree of acetalization (an amount of acetyl group) of the polyvinyl acetal resin is not particularly limited, but is preferably 0.0001 mol% or more and preferably 5 mol% or less.

The degree of acetal and degree of acetylation of the synthetic resin can be measured by ¹H-NMR (nuclear magnetic resonance spectrum).

Examples of the method for producing the polyvinyl acetal resin (modified polyvinyl acetal resin) include a method for acetalizing using a conventionally known method a polyvinyl alcohol obtained by saponifying polyvinyl acetate obtained by copolymerizing the monomer having an imine structure with vinyl acetate. In addition, a method may also be used for introducing an imine structure by acetalizing using a conventionally known method a polyvinyl alcohol having a structural unit having an amino group or an amide structure. A method may also be used for acetalizing using a conventionally known method a modified polyvinyl alcohol having an imine structure obtained by post-modifying a polyvinyl alcohol having a structural unit having an amino group or an amide structure. Furthermore, an imine structure may be introduced by post-modifying an unmodified polyvinyl acetal resin. In other words, the modified polyvinyl acetal resin may be an acetalized product of a polyvinyl alcohol having a structural unit having an amino group or an amide structure. Among them, a method is preferable for producing a modified polyvinyl acetal resin having an imine structure by acetalizing a polyvinyl alcohol having a structural unit having an amino group or an amide structure. In particular, when such a method is used, an imine structure can be obtained by adding excessive amounts of aldehyde and acid catalyst for use in acetalization.

In the method for excessively adding aldehyde, it is preferable to add 70 to 150 parts by weight aldehyde to 100 parts by weight a polyvinyl alcohol having a structural unit having an amino group or an amide structure. Particularly, as the aldehyde, acetaldehyde, propionaldehyde, n-butyraldehyde, isobutyraldehyde, n-valeraldehyde and phenylaldehyde are preferable.

In the method for excessively adding an acid catalyst, it is preferable to add the acid catalyst in an amount of 0.5% by weight or more with respect to the whole weight. In addition, it is preferable to add 5.0 to 70.0 parts by weight acid catalyst to 100 parts by weight a polyvinyl alcohol having a structural unit having an amino group or an amide structure. Particularly, as the acid catalyst, hydrochloric acid, nitric acid, sulfuric acid and para-toluenesulfonic acid are preferable.

The acetalization can be performed using a known method, and is preferably performed in an aqueous solvent, a mixed solvent of water and an organic solvent having compatibility with water, or an organic solvent. As the organic solvent compatible with water, for example, an alcohol-based organic solvent can be used. Examples of the organic solvent include alcohol-based organic solvents, aromatic organic solvents, aliphatic ester-based solvents, ketone-based solvents, lower paraffin-based solvents, ether-based solvents, amide-based solvents and amine-based solvents.

Examples of the alcohol-based organic solvent include methanol, ethanol, n-propanol, isopropanol, n-butanol and tert-butanol.

Examples of the aromatic organic solvent include xylene, toluene, ethylbenzene and methyl benzoate.

Examples of the aliphatic ester-based solvent include methyl acetate, ethyl acetate, butyl acetate, methyl propionate, ethyl propionate, methyl butyrate, ethyl butyrate, methyl acetoacetate and ethyl acetoacetate.

Examples of the ketone-based solvent include acetone, methyl ethyl ketone, methyl isobutyl ketone, cyclohexanone, methylcyclohexanone, benzophenone and acetophenone.

The lower paraffin-based solvents include hexane, pentane, octane, cyclohexane and decane.

The ether-based solvents include diethyl ether, tetrahydrofuran, ethylene glycol dimethyl ether, ethylene glycol diethyl ether and propylene glycol diethyl ether.

The amide-based solvents include N,N-dimethylformamide, N,N-dimethylacetamide, N-methylpyrrolidone and acetanilide.

The amine-based solvents include ammonia, trimethylamine, triethylamine, n-butylamine, di-n-butylamine, tri-n-butylamine, aniline, N-methylaniline, N,N-dimethylaniline and pyridine.

These organic solvents can be used alone or as a mixture of two or more solvents. Among them, ethanol, n-propanol, isopropanol and tetrahydrofuran are particularly preferable from the viewpoints of solubility in a resin and simplicity during purification.

The acetalization is preferably performed in the presence of an acid catalyst. The acid catalyst is not particularly limited, but includes mineral acids such as sulfuric acid, hydrochloric acid, nitric acid and phosphoric acid, carboxylic acids such as formic acid, acetic acid and propionic acid, and sulfonic acids such as methanesulfonic acid, ethanesulfonic acid, benzenesulfonic acid and para-toluenesulfonic acid. These acid catalysts may be used alone or in combination of two or more compounds. Among them, hydrochloric acid, nitric acid and sulfuric acid are preferable, and hydrochloric acid is particularly preferable.

As mentioned above, the polyvinyl acetal resin may be a graft copolymer with the vinyl compound.

The graft copolymer contains a graft copolymer having a "unit composed of polyvinyl acetal" and a "unit composed of a vinyl compound" (hereinafter, also simply referred to as "graft copolymer").

In the present invention, the "unit composed of polyvinyl acetal" and the "unit composed of a vinyl compound" refer to a unit composed of "polyvinyl acetal" and "a vinyl compound" present in the graft copolymer.

In addition, a graft copolymer having a unit composed of polyvinyl acetal and a unit composed of a vinyl compound refers to a branched copolymer in which, to a "unit composed of polyvinyl acetal" or a "unit composed of a vinyl compound" composing the main chain, a "unit composed of polyvinyl acetal" or a "unit composed of a vinyl compound" composing a side chain different from the main chain is bonded.

The molecular weight of the graft copolymer is not particularly limited, but it is preferable that the number average molecular weight (Mn) be 10,000 to 600,000, the weight average molecular weight (Mw) be 20,000 to 1,200,000 and the ratio (Mw/Mn) be 2.0 to 40. When the Mn, Mw and Mw/Mn are in such ranges, the strength of the scaffolding material for cell is suitably maintained.

The degree of acetalization in the graft copolymer can be measured by ¹H-NMR in which a soluble component of the graft copolymer in xylene is dissolved in deuterated dimethyl sulfoxide, for example.

The polyvinyl acetal resin preferably contains at least one structural unit selected from the group consisting of a structural unit having an imine structure, a structural unit having an amine structure and a structural unit having an amide structure in an amount of 0.1 mol% to 30 mol%, more preferably in an amount of 1 mol% to 10 mol%. In this case, the cell adhesion immediately after seeding can be enhanced.

The average degree of polymerization of a polyvinyl acetal resin when the polyvinyl acetal resin is used as the synthetic resin is preferably 100 or more, more preferably 200 or more, still more preferably 500 or more, especially preferably 1500 or more, and preferably 6000 or less, more preferably 3000 or less, still more preferably 2500 or less. When the average degree of polymerization is equal to or higher than the above-mentioned lower limit, the strength of the scaffolding material for cell culture can be suitably maintained even when swelled in a medium to be used for cell culture, so that the cell proliferation is improved. When the average degree of polymerization is equal to or lower than the above-mentioned upper limit, the handleability can be improved, so that the moldability of the scaffolding material for cell culture can be improved.

### (Other details of scaffolding material for cell culture)

The scaffolding material for cell culture according to the present invention is used for culturing cells. The scaffolding material for cell culture according to the present invention is used as a scaffold for cells when the cells are cultured. In the scaffolding material for cell culture according to the present invention, it is particularly preferable that a cell mass be seeded. However, in the scaffolding material for cell culture according to the present invention, a cell mass may not be seeded.

The cells include animal cells such as human, mouse, rat, pig, bovine and monkey cells. The cells also include somatic cells, stem cells, progenitor cells and differentiated cells. The somatic cell may also be cancer cells.

The differentiated cells include nerve cells, cardiomyocytes, retinal cells and hepatocytes.

The stem cells include pluripotent stem cells, tissue stem cells and tissue progenitor cells.

The tissue stem cells and tissue progenitor cells refer to cells having an ability to self-renew, belonging to any of the ectodermal, endodermal, mesodermal and germline tissues, and exhibiting a limited ability to differentiate into the constituent cell types of an organ to which they belong.

Examples of the tissue stem cells and tissue progenitor cells include neural stem cells, neural crest stem cells, retinal stem cells, corneal stem cells, keratinocyte epidermal stem cells, melanocyte stem cells, mammary gland stem cells, liver stem cells, intestinal stem cells, respiratory tract stem cells, hematopoietic stem cells, mesenchymal stem cells, cardiac stem cells, vascular endothelial progenitor cells, vascular pericytes, skeletal muscle stem cells, adipose stem cells, renal progenitor cells and sperm stem cells.

The cells are preferably somatic cells, stem cells, progenitor cells or differentiated cells. The somatic cells are preferably cancer cells, nerve cells, cardiomyocytes, retinal cells or hepatocytes.

### [Cell culture method]

The cell culture method according to the present invention is a culture method including using the scaffolding material for cell culture. Various cells can be cultured using the scaffolding material for cell culture according to the present invention. The cells include the above described cells. This is because the scaffolding material for cell culture according to the present invention is hardly swelled with the water in a culture medium, and thus can maintain so suitable hydrophilicity and strength that the fixation rate of cells after seeding is improved.

The cell culture method preferably includes a step of seeding a cell mass on the scaffolding material for cell culture. The cell mass can be obtained by adding a cell detaching agent to a confluent culture container and uniformly performing crushing by pipetting. The cell detaching agent is not particularly limited, but is preferably an ethylenediamine/phosphate buffer solution. The size of the cell mass is preferably 50 µm to 200 µm.

### [Container for cell culture]

The present invention also relates to a container for cell culture including the scaffolding material for cell culture. In other words, the present invention relates to a container for cell culture, the container for cell culture including the cell scaffold material on at least a part of a cell culture region. In the container for cell culture, the scaffolding material for cell culture is preferably in the form of a film, and is preferably a resin film. The container for cell culture includes a container body and the scaffolding material for cell culture (resin film) arranged on the surface of the container body.

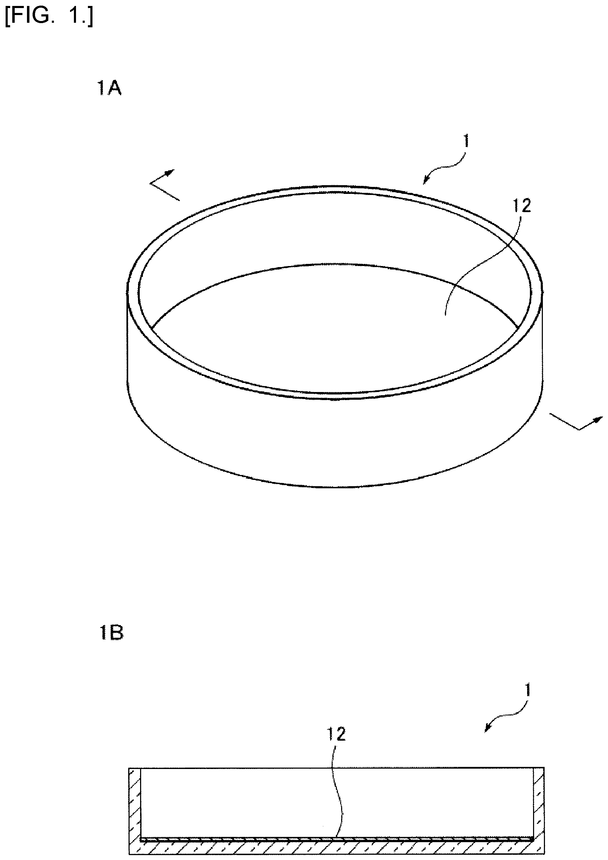

Fig. 1A is a schematic perspective view of the container for cell culture according to an embodiment, and Fig. 1B is a schematic sectional side view thereof. The container for cell culture is not particularly limited for shape, but examples thereof include an aspect in which a bottomed cylindrical Petri dish 1 as shown in Fig. 1A is prepared, and a resin film 12 is provided on a cell culture region on the bottom surface as shown in Fig. 1B.

In cell culture, the scaffolding material for cell culture can be used not only for planar culture (two-dimensional culture method) but also for culturing cells on a base material in a state closer to an in-vivo state, such as a porous membrane or a hydrogel (three-dimensional culture method). This is because cells can be efficiently proliferated by using the scaffolding material for cell culture in a bioreactor or the like.

The scaffolding material for cell culture is preferably used in a two-dimensional culture method because it has suitable hydrophilicity and strength.

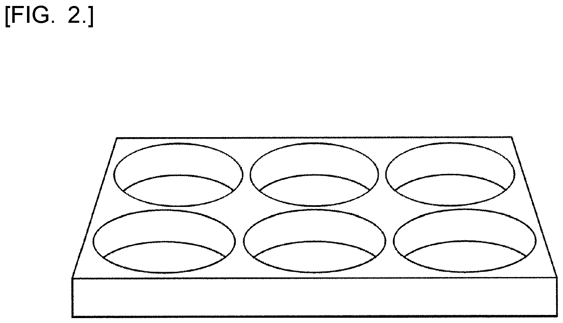

The container for planar culture (two-dimensional culture method) is not particularly limited for shape and size, but includes a test plate for cell culture having one or more wells (holes) and a flask for cell culture. For example, a container for cell culture having six wells as shown in Fig. 2 can be used. The number of wells in the microplate is not limited, but includes, for example, 2, 4, 6, 12, 24, 48, 96 and 384.

The shape of the well is not particularly limited, but includes, for example, a perfect circle, ellipse, triangle, square, rectangle, and pentagon. The shape of the bottom surface of the well is not particularly limited, but includes a flat bottom, a round bottom and irregularities.

The material of the test plate for cell culture having one or more wells (holes) or the material of the flask for cell culture are not particularly limited, but includes a polymer resin, metal and inorganic material. The polymer resin includes polystyrene, polyethylene, polypropylene, polycarbonate, polyester, polyisoprene, cycloolefin polymer, polyimide, polyamide, polyamideimide, (meth)acrylic resin, epoxy resin and silicone. The metal includes stainless steel, copper, iron, nickel, aluminum, titanium, gold, silver and platinum. The inorganic material includes silicon oxide (glass), aluminum oxide, titanium oxide, zirconium oxide, iron oxide and silicon nitride.

In addition to the above, the scaffolding material for cell culture can be used in a suspension culture method in which cells are freely suspended and grown in a medium.

### [Other embodiments]

In addition to the scaffolding material for cell culture, the present invention provides an invention using the scaffolding material for cell culture as another embodiment.

For example, in the present invention, a carrier (medium) for cell culture containing the scaffolding material for cell culture and a polysaccharide is provided. Various polysaccharides can be used as the polysaccharide without any particular limitation. Among them, water-soluble polysaccharides are preferable.

In addition, the present invention provides a fiber for cell culture including the scaffolding material for cell culture. In this case, it is preferable that the scaffolding material for cell culture be applied on the fiber. In addition, the scaffolding material for cell culture may be in a form impregnated or kneaded in the fiber. The fiber for cell culture is suitable for a three-dimensional culture method for cells that are difficult to adhere to a planar structure such as a flask, but easily adhere to a three-dimensional structure such as a fibril-like structure.

The scaffolding material for cell culture may be cross-linked. This is because crosslinking can suppress water swelling and suitably increase the strength. Using a crosslinking agent can provide the crosslinked scaffolding material for cell culture.

The crosslinking agent is not particularly limited, but includes polyalcohol, polycarboxylic acid, hydroxycarboxylic acid, metal soap and polysaccharides.

The polyalcohol is not particularly limited, but includes ethylene glycol, propylene glycol, butanediol, pentanediol, hexanediol, heptanediol, octanediol, nonanediol, decanediol, dodecanediol, undecanediol, diethylene glycol, triethylene glycol, tetraethylene glycol, polyethylene glycol, catechol, pyrogallol, diboronic acid, methylenediboronic acid, ethylenediboronic acid, propylene diboronic acid, phenylenediboronic acid, biphenyldiboronic acid and bisphenol derivatives.

The polycarboxylic acid is not particularly limited, but includes oxalic acid, malonic acid, succinic acid, glutaric acid, adipic acid, pimelic acid, suberic acid, azelaic acid, sebacic acid, phthalic acid and poly(meth)acrylic acid.

The hydroxycarboxylic acid is not particularly limited, but includes glycolic acid, lactic acid, tartronic acid, glyceric acid, hydroxybutyric acid, malic acid, tartaric acid, cytomaric acid, citric acid, isocitric acid, leucic acid, mevalonic acid, pantoic acid, ricinoleic acid, ricineraidic acid, cerebronic acid, quinic acid, shikimic acid, hydroxybenzoic acid, salicylic acid, creosoteic acid, vanillic acid, syringic acid, pyrocatechuic acid, resorcylic acid, protocatechuic acid, gentisic acid, orsellinic acid, gallic acid, mandelic acid, benzilic acid, atrolactic acid, melilotic acid, phloretic acid, coumaric acid, umbellic acid, caffeic acid, ferulic acid, sinapinic acid and hydroxystearic acid.

The metal soap is not particularly limited, but includes salts of fatty acids such as stearic acid, lauric acid, ricinoleic acid and octylic acid with metals such as lithium, sodium, magnesium, calcium, barium, zinc and aluminum.

The polysaccharides are not particularly limited, but include pectin, guar gum, xanthan gum, tamarind gum, carrageenan, propylene glycol, carboxymethylcellulose, amylose, amylopectin, glycogen, cellulose, chitin, agarose, carrageenan, heparin, hyaluronic acid, xyloglucan and glucomannanic acid.

### EXAMPLES

Hereinafter, a description is made of the present invention with reference to Examples and Comparative Examples, but the present invention is not limited to the following Examples. The content of the structural units (mol%) having an amine structure, an imine structure and an amide structure, degree of acetalization (mol%), amount of acetyl group (mol%) and amount of hydroxyl group (mol%) in an obtained synthetic resin were measured by dissolving the synthetic resin in DMSO-d6 (dimethyl sulfoxide) and using ¹H-NMR (nuclear magnetic resonance spectrum).

### [Example 1]

### (Preparation of polyvinyl butyral resin)

A reactor equipped with a stirrer was charged with 2,700 mL of ion-exchanged water and 300 g of polyvinyl alcohol containing 2 mol% structural unit having an amino group represented by the formula (21) (having an average degree of polymerization of 250 and a degree of saponification of 97 mol%), followed by dissolution by heating with stirring to prepare a solution. Next, to the solution, 35% by mass hydrochloric acid as a catalyst was added such that the concentration of hydrochloric acid became 0.2% by mass, and after the temperature was adjusted to 15°C, 22 g of n-butyraldehyde (n-BA) was added while being stirred. Thereafter, when 148 g of n-butyraldehyde (n-BA) was added, polyvinyl butyral was precipitated in the form of white particles. Fifteen minutes after the precipitation, 35% by mass hydrochloric acid was added such that the concentration of hydrochloric acid became 1.8% by mass, and the mixture was heated to 550°C, and then aged at 50°C for 2 hours. Next, the solution was cooled and neutralized, and then the polyvinyl butyral was washed with water and dried to yield a polyvinyl butyral resin.

The obtained polyvinyl butyral resin had an average degree of polymerization of 250, an amount of hydroxyl group of 20 mol%, an amount of acetyl group of 1 mol% and a degree of acetalization of 77 mol%.

The elemental composition of the obtained resin was analyzed by X-ray photoelectron spectroscopy (apparatus: multifunctional scanning X-ray photoelectron spectrometer (XPS), PHI 5000 VersaProbe III, manufactured by ULVAC-PHI, Inc.). Then, the total amount of all the detected elements was defined as 100%, and the occupation rate (%) of nitrogen in all the elements was defined as the nitrogen content.

### (Preparation of container for cell culture)

By dissolving 1 g of the obtained polyvinyl butyral resin in 19 g of butanol, a solution of polyvinyl butyral resin was obtained. By discharging 150 µL of the obtained solution of polyvinyl butyral resin onto a ϕ22 mm cover glass (manufactured by Matsunami Glass Ind., Ltd., 22 round No. 1 was used after dust was removed with air duster) and spinning it at 2,000 rpm for 20 seconds using a spin coater, a smooth resin film was obtained. By placing the obtained resin film on a ϕ22 mm polystyrene dish together with the cover glass, a container for cell culture was obtained.

Using the obtained container for cell culture, tests were conducted under the following conditions.

### (Method for cell culture test)

To the obtained container for cell culture, 1 mL of phosphate buffered saline was added, and the mixture was allowed to stand for 1 hour in an incubator at 37°C. After removing the phosphate buffered saline in the dish, 3×10⁴ cells of human fresh hepatocytes derived from a chimeric mouse (PXB-cells manufactured by PhoenixBio Co., Ltd.) were seeded. Next, 1 mL of medium RM-101 (manufactured by Toyo Gosei Co., Ltd.) was added, followed by culture in an incubator at 37°C with a CO₂ concentration of 5%.

### (Evaluation)

### (1) Initial adhesion (fixation after seeding)

In the cell culture test, cells were detached using trypsin 24 hours after seeding of the cells. The number of cells was measured using an auto cell counter (Auto Cell Counter EVE, manufactured by NanoEnteck Inc.).

### <Evaluation criteria for initial adhesion>

○○○: The number of cells is 2.5 × 10⁴ cells or more
○○: The number of cells is 1.5 × 10⁴ cells or more and less than 2.5 × 10⁴ cells
○: The number of cells is 1.0 × 10⁴ cells or more and less than 1.5 × 10⁴ cells
×: The number of cells is less than 1.0 × 10⁴ cells

### (2) Cell proliferation

In the cell culture test, cells were detached using trypsin 3 days after seeding of the cells. The number of cells was measured using an auto cell counter (Auto Cell Counter EVE, manufactured by NanoEnteck Inc.).

### <Evaluation criteria for cell proliferation>

○○○: The number of cells is 2.0 × 10⁵ cells or more
○○: The number of cells is 1.0 × 10⁵ cells or more and less than 2.0 × 10⁵ cells
○: The number of cells is 5.0 × 10⁴ cells or more and less than 1.0 × 10⁵ cells
×: The number of cells is less than 5.0 × 10⁴ cells

### [Example 2]

The test was performed in the same manner as in Example 1 except that a polyvinyl alcohol containing 2 mol% structural unit having an amino group represented by the formula (21) having an average degree of polymerization of 1,600 and a degree of saponification of 97 mol% was used, instead of the polyvinyl alcohol containing 2 mol% structural unit having an amino group represented by the formula (21) (having an average degree of polymerization of 250 and a degree of saponification of 97 mol%).

### [Example 3]

A polyvinyl acetal was obtained in the same manner as in Example 1 except that a polyvinyl alcohol having an average degree of polymerization of 250 and a degree of saponification of 97 mol% was used, instead of the polyvinyl alcohol containing 2 mol% structural unit having an amino group represented by the formula (21) (having an average degree of polymerization of 250 and a degree of saponification of 97 mol%). In 500 parts by weight tetrahydrofuran, 100 parts by weight obtained polyvinyl acetal having a degree of polymerization of about 250 and 1 part by weight N-vinylpyrrolidone were dissolved to prepare a resin solution. In the prepared resin solution, 0.05 parts by weight Irgacure184 (manufactured by BASF) was dissolved, and the resultant mixture was applied onto a PET film. The coated product was irradiated with light having a wavelength of 365 nm at an integrated light amount of 2000 mJ/cm² using a UV conveyor device "ECS301G1" manufactured by Eye Graphics Co., Ltd. at 25°C to prepare a composite resin solution. The prepared composite resin solution was vacuum-dried at 80°C for 3 hours to prepare a composite resin. The prepared resin was measured for weight average molecular weight in terms of polystyrene by GPC method using "2690 Separations Model" manufactured by Waters Corporation as a column. The weight average molecular weight was about 40,000. The prepared composite resin was adjusted to a 3% by weight butanol solution, and the test was conducted in the same manner as in Example 1.

### [Example 4]

The test was performed in the same manner as in Example 3 except that 10 parts by weight N-vinylpyrrolidone was added to 100 parts by weight polyvinyl acetal. The weight average molecular weight of the obtained resin was about 60,000.

### [Example 5]

The test was performed in the same manner as in Example 3 except that 30 parts by weight N-vinylpyrrolidone was added to 100 parts by weight polyvinyl acetal. The weight average molecular weight of the obtained resin was about 50,000.

### [Example 6]

The test was performed in the same manner as in Example 3 except that 2 parts by weight N-isopropylacrylamide was added to 100 parts by weight polyvinyl acetal. The weight average molecular weight of the obtained resin was about 60,000.

### [Example 7]

The test was performed in the same manner as in Example 3 except that 2 parts by weight polyamide was added to 100 parts by weight polyvinyl acetal. The weight average molecular weight of the obtained resin was about 60,000.

### [Example 8]

The test was performed in the same manner as in Example 3 except that 2 parts by weight poly-L-lysine was added to 100 parts by weight polyvinyl acetal. The weight average molecular weight of the obtained resin was about 50,000.

### [Example 9]

In 300 parts by weight tetrahydrofuran, 5 parts by weight N-vinylpyrrolidone, 15 parts by weight butyl methacrylate, 30 parts by weight ethyl acrylate and 50 parts by weight methyl methacrylate were dissolved to prepare an acrylic monomer solution. In the prepared acrylic monomer solution, 2 parts by weight Irgacure184 (manufactured by BASF) was dissolved, and the resultant mixture was applied onto a PET film. The coated product was irradiated with light having a wavelength of 365 nm at an integrated light amount of 2,000 mJ/cm² using a UV conveyor device "ECS301G1" manufactured by Eye Graphics Co., Ltd. at 25°C to prepare an acrylic resin solution. The prepared acrylic resin solution was vacuum-dried at 80°C for 3 hours to prepare an acrylic resin. The prepared acrylic resin was adjusted to a 3% by weight butanol solution, and the test was conducted in the same manner as in Example 1. The weight average molecular weight of the obtained resin was about 90,000.

### [Comparative Example 1]

The test was performed in the same manner as in Example 1 using only a polystyrene dish without using the scaffolding material resin.

### [Comparative Example 2]

The test was performed in the same manner as in Example 1 except that the addition amounts of n-butyraldehyde was changed to 22 g and 88 g instead of 22 g and 148 g in Example 1.

### [Comparative Example 3]

The test was performed in the same manner as in Example 1 except that polyvinyl alcohol having an average degree of polymerization of 1,000 and a degree of saponification of 98 mol% was used as the synthetic resin.

### [Comparative Example 4]

A polyacrylamide resin was obtained by mixing 100 parts by weight N-isopropylacrylamide, 75 parts by weight ethyl acetate and 0.5 parts by weight azobisisobutyronitrile, followed by polymerization at 65°C for 8 hours under a nitrogen atmosphere. The prepared resin was measured for weight average molecular weight in terms of polystyrene by GPC method using "2690 Separations Model" manufactured by Waters Corporation as a column. The weight average molecular weight was about 90,000 (the degree of polymerization was about 800). The test was performed in the same manner as in Example 1 except that the obtained polyacrylamide resin was used as the synthetic resin.

### [Comparative Example 5]

The test was performed in the same manner as in Comparative Example 4 except that 100 parts by weight ethyl acrylate was used instead of 100 parts by weight N-isopropylacrylamide. The weight average molecular weight of the obtained resin was about 60,000.

### [Comparative Example 6]

The test was performed in the same manner as in Comparative Example 4 except that 100 parts by weight butyl methacrylate was used instead of 100 parts by weight N-isopropylacrylamide. The weight average molecular weight of the obtained resin was about 80,000.

### [Comparative Example 7]

The test was performed in the same manner as in Example 3 except that 70 parts by weight N-vinylpyrrolidone was added to 30 parts by weight polyvinyl acetal. The weight average molecular weight of the obtained resin was about 90,000.

The obtained results are summarized in Tables 1 and 2.

**[Table 1]**

| | | Ex. 1 | Ex. 2 | Ex. 3 | Ex. 4 | Ex. 5 | Ex. 6 | Ex. 7 | Ex. 8 | Ex. 9 |
|---|---|---|---|---|---|---|---|---|---|---|
| Polyvinyl acetal resin | Degree of acetalization (mol%) | 77 | 76 | 70 | 65 | 54 | 70 | 70 | 70 | - |
| | Amount of acetyl group (mol%) | 1 | 1 | 1 | 1 | 1 | 1 | 1 | 1 | - |
| | Amount of hydroxyl group (mol%) | 20 | 21 | 28 | 25 | 21 | 27 | 27 | 27 | - |
| | Content of structural unit having amine structure (1) (mol%) | 0.3 | 0.3 | - | - | - | - | - | 2 | - |
| | Content of structural unit having imine structure (2) (mol%) | 1.7 | 1.7 | - | - | - | - | - | - | - |
| | Content of structural unit having amide structure (3) (mol%) | - | - | 1 | 9 | 24 | 2 | 2 | - | - |
| | Total content of structural unit having amine structure, structural unit having imine structure and structural unit having amide structure ((1) + (2) + (3)) (mol%) | 2 | 2 | 1 | 9 | 24 | 2 | 2 | 2 | - |
| Poly(meth)acrylic ester | Content of structural unit having amine structure (1) (mol%) | - | - | - | - | - | - | - | - | 5 |
| | Content of structural unit having amide structure (3) (mol%) | - | - | - | - | - | - | - | - | - |
| | Total content of structural unit having amine structure and structural unit having amide structure ((1) + (3)) (mol%) | - | - | - | - | - | - | - | - | 5 |
| | Content of structural unit derived from butyl methacrylate (mol%) | - | - | - | - | - | - | - | - | 11 |
| | Content of structural unit derived from ethyl acrylate (mol%) | - | - | - | - | - | - | - | - | 31 |
| | Content of structural unit derived from methyl methacrylate (mol%) | - | - | - | - | - | - | - | - | 53 |
| Properties of resin | Average degree of polymerization | 250 | 1600 | 250 | 250 | 250 | 250 | 250 | 250 | 800 |
| | Nitrogen content (% by mass) | 0.7 | 0.7 | 0.5 | 3.5 | 8.1 | 0.6 | 0.6 | 0.7 | 0.9 |
| Evaluation for culture | Initial adhesion | ○○ | ○○ | ○○○ | ○○○ | ○○ | ○ | ○ | ○ | ○ |
| | Cell proliferation | ○○ | ○○ | ○○○ | ○○○ | ○○ | ○ | ○ | ○ | ○ |

**[Table 2]**

| | | Comp.Ex. 1 | Comp.Ex. 2 | Comp.Ex. 3 | Comp.Ex. 4 | Comp.Ex. 5 | Comp.Ex. 6 | Comp.Ex. 7 |
|---|---|---|---|---|---|---|---|---|
| Polyvinyl acetal resin | Degree of acetalization (mol%) | - | 40 | 0 | - | - | - | 21 |
| | Amount of acetyl group (mol%) | - | 3 | 2 | - | - | - | 0 |
| | Amount of hydroxyl group (mol%) | - | 57 | 98 | - | - | - | 8 |
| | Content of structural unit having amine structure (1) (mol%) | - | - | - | - | - | - | - |
| | Content of structural unit having imine structure (2) (mol%) | - | - | - | - | - | - | - |
| | Content of structural unit having amide structure (3) (mol%) | - | - | - | - | - | - | 71 |
| | Total content of structural unit having amine structure, structural unit having imine structure and structural unit having amide structure ((1) + (2) + (3)) (mol%) | - | - | - | - | - | - | 71 |
| Poly(meth)acrylic ester | Content of structural unit having amine structure (1) (mol%) | - | - | - | - | - | - | - |
| | Content of structural unit having amide structure (3) (mol%) | - | - | - | 100 | - | - | - |
| | Total content of structural unit having amine structure and structural unit having amide structure ((1) + (3)) (mol%) | - | - | - | 100 | - | - | - |
| | Content of structural unit derived from butyl methacrylate (mol%) | - | - | - | - | - | 100 | - |
| | Content of structural unit derived from ethyl acrylate (mol%) | - | - | - | - | 100 | - | - |
| | Content of structural unit derived from methyl methacrylate (mol%) | - | - | - | - | - | - | - |
| Properties of resin | Average degree of polymerization | - | 250 | 1000 | 800 | 600 | 550 | 250 |
| | Nitrogen content (% by mass) | 0 | 0 | 0 | 12.2 | 0 | 0 | 10.2 |
| Evaluation for culture | Initial adhesion | × | × | × | × | × | × | × |
| | Cell proliferation | × | × | × | × | × | × | × |

### EXPLANATION OF SYMBOLS

- 1:: Petri dish
- 12:: Resin film

## Claims

1. A scaffolding material for cell culture,
the scaffolding material for cell culture comprising a synthetic resin, and being used for culturing a progenitor cell or a differentiated cell,
the synthetic resin being a poly(meth)acrylic ester or a polyvinyl acetal resin, and having
a nitrogen content of the synthetic resin of 0.1% by mass or more and 10% by mass or less.

2. The scaffolding material for cell culture according to claim 1, wherein the synthetic resin includes a Bronsted basic group in an amount of 1 mol% or more and 50 mol% or less.

3. The scaffolding material for cell culture according to claim 2, wherein the Bronsted basic group is an amine-based basic group.

4. The scaffolding material for cell culture according to claim 2 or 3, wherein the synthetic resin contains at least one structural unit selected from the group consisting of a structural unit having an amine structure, a structural unit having an imine structure and a structural unit having an amide structure.

5. The scaffolding material for cell culture according to any one of claims 1, 2 and 4, wherein the synthetic resin contains a structural unit having an amide structure.

6. The scaffolding material for cell culture according to any one of claims 1, 2 and 4, wherein the synthetic resin contains both of a structural unit having an imine structure and a structural unit having an amine structure.

7. The scaffolding material for cell culture according to any one of claims 1 to 6, wherein the synthetic resin contains a polyvinyl acetal resin having a degree of acetalization of 54 mol% or more.

8. The scaffolding material for cell culture according to any one of claims 1 to 6, wherein the synthetic resin contains a polyvinyl butyral resin.

9. A container for cell culture, comprising the scaffolding material for cell culture according to any one of claims 1 to 8.

10. A carrier for cell culture, comprising:
the scaffolding material for cell culture according to any one of claims 1 to 8; and
a polysaccharide.

11. A fiber for cell culture, comprising the scaffolding material for cell culture according to any one of claims 1 to 8.

12. A method for culturing a cell, comprising using the scaffolding material for cell culture according to any one of claims 1 to 8.
